(19)

**Europäisches Patentamt**

**European Patent Office**

**Office européen des brevets**

(11) **EP 4 782 552 A1**

(12) **EUROPEAN PATENT APPLICATION**

(43) Date of publication:
29.07.2026 Bulletin 2026/31

(21) Application number: 25382052.6

(22) Date of filing: **28.01.2025**

(51) International Patent Classification (IPC):
**C12Q 1/6883** (2018.01)    **C12N 5/077** (2010.01)

(52) Cooperative Patent Classification (CPC):
**C12Q 1/6883; C12N 5/0654;** C12Q 2600/156

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **Fundación Pública Galega Instituto de Investigación Sanitaria de Santiago de Compostela**
  **15706 Santiago de Compostela A Coruña (ES)**
- **Servizo Galego De Saúde**
  **15703 Santiago de Compostela, A Coruna (ES)**

(72) Inventors:
- **GÓMEZ VAAMONDE, Rodolfo**
  **15706 Santiago de Compostela (ES)**

- **PIÑEIRO RAMIL, María**
  **15706 Santiago de Compostela (ES)**
- **ALONSO PÉREZ, Ana**
  **15706 Santiago de Compostela (ES)**
- **GUILLÁN FRESCO, María**
  **15706 Santiago de Compostela (ES)**
- **PAZOS PÉREZ, Andrés**
  **15706 Santiago de Compostela (ES)**
- **LÓPEZ FAGÚNDEZ, Miriam**
  **15706 Santiago de Compostela (ES)**
- **JORGE MORA, Alberto Agustín**
  **15006 A Coruña (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

(54) **DIAGNOSIS, PROGNOSIS, PREVENTION AND/OR TREATMENT OF HETEROTOPIC OSSIFICATION**

(57)    The present invention refers to a method for the diagnosis, prognosis, prevention and/or treatment of heterotopic ossification (HO). On the other hand, the present invention also refers to a method for promoting osteogenesis, promoting chondrogenesis and/or inhibiting adipogenesis.

EP 4 782 552 A1

**Description**

**FIELD OF THE INVENTION**

The present invention refers to the medical field. Particularly, the present invention refers to a method for the diagnosis, prognosis, prevention and/or treatment of heterotopic ossification (HO). On the other hand, the present invention also refers to a method for promoting osteogenesis, promoting chondrogenesis and/or inhibiting adipogenesis.

**STATE OF THE ART**

Progressive Osseous Heteroplasia (POH) (OMIM 166350) is an ultra-rare genetic disease characterized by HO of the skin, subcutaneous fat, and skeletal muscle. POH is distinguished clinically from other related genetic disorders (Albright hereditary dystrophy (AHO), pseudohypoparathyroidism (PHP), pseudopseudohypoparathyroidism (PPHP), and primary osteoma cutis) by the deep and progressive nature of the ossifications in the absence of hormone resistance or other AHO features (i.e., short stature, obesity, round face, brachydactyly, and neurobehavioral problems). Most POH patients start developing ectopic bone before one year of age, which causes chronic pain and joint ankylosis, and some of them require surgery or even amputation during childhood or puberty. Nowadays, the only treatment option for these patients is the surgical resection of ossifications, which usually leads to their recurrence.

Most cases of POH are caused by heterozygous inactivating mutations in GNAS, a complex locus which encodes several transcripts with unique promoters and first exons and common downstream exons. Adding yet another level of complexity, the expression of GNAS transcripts is regulated by genomic imprinting, with some of the alternative first exons being transcribed exclusively from the maternal or paternal allele. The main product of this locus is the alpha subunit of the G-stimulatory protein of adenylyl cyclase (Gsα), which is expressed biallelically in most tissues. Gsα is ubiquitously expressed and activated by many G proteincoupled receptors (GPRCs), many of which play critical roles in bone development and remodelling. As a result of GNAS inactivation, less adenylyl cyclase is activated upon ligand binding to receptors, and thus less cyclic adenosine monophosphate (cAMP) is produced. Importantly, inhibition of cAMP signalling has been demonstrated to enhance osteoblast differentiation.

Nonetheless, the presence of a specific GNAS mutation does not predict a specific disorder, phenotype, or severity of progression. For instance, the same heterozygous 4-base pair deletion has been associated with different phenotypes of AHO, PHP, PPHP, and POH. Strikingly, this specific mutation has been found in both obese PHP/PPHP patients and POH patients, who are never obese. Interestingly, ossifications in POH patients manifest in a mosaic pattern, following a dermomyotomal distribution, which has been suggested to be the consequence of loss of heterozygosity at the GNAS locus. However, even though heterozygous mutations in a mosaic state with homozygous wild-type GNAS have been identified in POH patients and asymptomatic parents, the loss of the wild-type allele has never been found in clinical samples.

GNAS loss-of-function mutations affect numerous signalling pathways, including key regulators of skeletal maturation and regeneration, such as Wingless-related integration site (Wnt) and Hedgehog (Hh), ultimately leading to aberrant upregulation of bone-forming genes. The development of HO results from the pathological differentiation of progenitor cells, which is promoted by inflammatory signals and growth factors such as interleukin-1β (IL-1β) and transforming growth factor β1 (TGFβ1). During the formation of ectopic bone, proteomic biomarkers of HO, such as alkaline phosphatase (ALPL) and osteopontin/secreted phosphoprotein-1 (SPP1), are markedly increased. Nonetheless, the molecular mechanisms underlying the progression of this disease remain poorly understood. Likewise, the cellular origin of HO has yet to be elucidated, and whether it derives from tissue-resident or circulating progenitors remains controversial. Emerging evidence indicates that peripheral nerve-derived cells may contribute to this pathological process by differentiating into transient brown adipocyte-like cells and early chondrocytes upon bone morphogenetic protein 2 (BMP2) stimulation. Within the HO microenvironment, these brown adipocyte-like cells regulate oxygen tension, which is critical for chondrogenesis and vascularization.

There is an unmet medical need of finding reliable methods for the diagnosis, prognosis, prevention and/or treatment of HO. For instance, due to the low incidence of POH, samples from POH patients are scarce, and there are no available cell lines with the specific mutation and epigenetic context that cause this disease. With the current technology, it is not possible to reproduce this mutation while keeping the genetic, epigenetic, and tissue context of the pathology. This lack of research tools has hampered the development of new pharmacological treatments for these patients, who suffer from chronic pain and severe disability from infancy.

The present invention is focused on solving this problem and a reliable method for the diagnosis, prognosis, prevention and/or treatment of HO is herein provided. On the other hand, the present invention also provides a method for promoting osteogenesis, promoting chondrogenesis and/or inhibiting adipogenesis.

**DESCRIPTION OF THE INVENTION**

## Brief description of the invention

The present invention refers to a method for the diagnosis, prognosis, prevention and/or treatment of HO. On the other hand, the present invention also refers to a method for promoting osteogenesis, promoting chondrogenesis and/or inhibiting adipogenesis.

Particularly, the inventors of the present invention developed two osteoblast-like cell lines from a POH patient (an ultra-rare genetic disease characterized by HO of the skin, subcutaneous fat, and skeletal muscle) with a heterozygous GNAS loss-of-function mutation. The characterization of these cell lines surprisingly revealed that GNAS wild-type allele was missing in one of them, and subsequent analysis of the patient's tissues confirmed the presence of GNAS double-mutant cells in varying proportions, with up to 25% in nervous tissues. GNAS double-mutant cells exhibited an aggressive phenotype, with high expression of genes related to POH pathogenesis, even in a pro-adipogenic environment. Furthermore, GNAS double-mutant cells inhibited adipogenesis and promoted osteochondrogenesis in wild-type cells through paracrine signalling. So, the present invention describes, for the first time, the presence of GNAS double-mutant cells in a POH patient and discovered that these cells may have important implications for the severity of the pathology.

Such as it is shown in the results provided in the Examples below, the inventors of the present invention have identified the loss of GNAS wild-type allele in ectopic bone and surrounding tissues, that biallelic loss of GNAS increases the expression of bone and cartilage-related genes, that GNAS double-mutant cells inhibit adipogenesis, induce osteochondrogenesis and promote inflammation through paracrine signalling, that silencing of GNAS inhibits adipogenesis and induces osteochondrogenesis, and that transcriptomic analysis of double-mutant cells reveals enrichment of genes related to bone formation and inflammation.

So, the first embodiment of the present invention refers to an *in vitro* method for the diagnosis and/or prognosis of HO which comprises assessing the presence of cells (hereinafter *cells of the invention* or $\alpha2$) characterized in that they comprise a GNAS loss-of-function mutation and lack the GNAS wild-type allele, in a biological sample obtained from the subject, wherein the presence of said cells is indicative that the subject may be suffering from HO.

Detecting a loss-of-function mutation often involves identifying a mutation that disrupts the normal function of a gene. The detection of a loss-of-function mutation can be carried out by the person skilled in the art following methods which belong to the common general knowledge, for instance:

- DNA-Level Analysis:

    a) Sequencing-Based Methods:

        a. Sanger Sequencing: Useful for small regions of DNA or single genes. It identifies point mutations, insertions, and deletions.
        b. Next-Generation Sequencing (NGS): High-throughput method to identify LoF mutations across the genome or exome, such as nonsense mutations, frameshifts, or large deletions.
        c. Long-Read Sequencing: Identifies structural variants, large indels, or complex mutations that disrupt gene function.

    b) PCR-Based Methods:

        a. Allele-Specific PCR: Detects specific known loss-of-function mutations.
        b. Quantitative PCR (qPCR): Detects deletions or duplications in the gene of interest.

    c) Copy Number Variation (CNV) Analysis:

        a. Techniques like comparative genomic hybridization (CGH) or digital droplet PCR assess for large deletions or duplications leading to loss of gene function.

- RNA-Level Analysis:

    a) RT-qPCR: Measures gene expression levels; a loss-of-function mutation often results in reduced or absent mRNA.
    b) RNA Sequencing (RNA-seq): Identifies aberrant splicing (e.g., exon skipping, intron retention) caused by splice-site mutations.

- Protein-Level Analysis:

a) Western Blotting: Detects absence or truncation of protein caused by a loss-of-function mutation.
b) Immunohistochemistry (IHC): Assesses protein localization and expression in tissue samples.

- Functional Assays:

a) Enzyme Activity Assays: Measure the enzymatic function of the gene product to identify functional loss.
b) Reporter Assays: Use reporter constructs to determine the functional impact of mutations on gene regulation.
c) CRISPR Screening: Introducing targeted mutations to validate loss-of-function effects in cell lines.

- Structural Prediction and In Silico Tools:

a) Pathogenicity Predictors: Tools like PolyPhen, SIFT, and MutationTaster predict the impact of variants on protein function.
b) Protein Modeling: Structural prediction to assess disruptions in protein folding or active sites.

- Phenotypic Correlation:

a) Knockout Models: Generate organism or cell models (e.g., using CRISPR) to confirm LoF through phenotypic analysis.
b) Clinical Correlation: Study phenotypes in patients with identified mutations (e.g., family studies).

On the other hand, assessing whether the wild-type allele has been lost or is missing typically involves molecular biology techniques that detect or quantify the presence of specific genetic sequences. The assessment of whether a wild-type allele has been lost or is missing can be carried out by the person skilled in the art following methods which belong to the common general knowledge, for instance:

- Polymerase Chain Reaction (PCR):

a) Conventional PCR: Primers designed for the wild-type allele can indicate its presence or absence by amplifying the target sequence. A lack of amplification suggests the allele is missing.
b) Quantitative PCR (qPCR): Measures the abundance of the wild-type allele relative to a reference gene. A significant reduction in signal can indicate loss.
c) Allele-Specific PCR: Utilizes primers specific to the wild-type sequence to differentiate it from mutated or edited alleles.

- Droplet Digital PCR (ddPCR):
d) Allows precise quantification of the wild-type allele by partitioning the PCR reaction into thousands of droplets. Each droplet acts as an individual reaction chamber, increasing sensitivity for detecting loss of the allele.

- Southern Blotting:
e) Involves digesting genomic DNA, separating fragments by gel electrophoresis, transferring to a membrane, and hybridizing with a probe specific to the wild-type allele. The absence of the expected band can indicate loss.

- Next-Generation Sequencing (NGS):

f) Whole-Genome Sequencing (WGS): Identifies the presence or absence of the wild-type allele across the genome.
g) Targeted Sequencing: Focuses on the region of interest to determine if the wild-type allele is still present.
h) Copy Number Variation Analysis: Detects loss of genetic material, including the wild-type allele.

- Fluorescence In Situ Hybridization (FISH):
i) Uses fluorescently labelled probes to bind specific DNA sequences in chromosomes. If the wild-type allele is missing, the probe will fail to hybridize to that location.

- CRISPR/Cas9-Based Reporters:
j) Constructs that link Cas9 activity to a reporter gene can indicate the presence or absence of the wild-type allele when combined with specific guide RNAs targeting it.

- Immunoassays:
  k) If the wild-type allele encodes a protein, its loss can sometimes be inferred through assays such as Western blotting or ELISA to detect protein expression.

- Loss of Heterozygosity (LOH) Assays:
  l) Detects the loss of one allele (e.g., the wild type) in heterozygous conditions by comparing the allelic balance in a sample.

- Single-Nucleotide Polymorphism (SNP) Analysis:
  m) SNP genotyping can detect the presence or absence of specific alleles if the wild-type allele has a unique SNP signature.

The above-mentioned techniques may require, as a first step, isolating the cells and/or prepare a calibration curve.

So, the first embodiment is clear since the person skilled in the art could identify, with the above methods, the presence of cells characterized in that they comprise a GNAS loss-of-function mutation and lack the GNAS wild-type allele.

The second embodiment of the present invention refers to GNAS protein, gene editing tool, or gene tool for increasing the expression of GNAS gene, for use in a method for the treatment and/or prevention of HO wherein the method comprises reducing the amount or eliminating cells comprising a GNAS loss-of-function mutation and lacking the GNAS wild-type allele. Alternatively, this embodiment refers to a method for the treatment and/or prevention of HO which comprises the administration of a therapeutically effective amount of GNAS protein or the use of gene editing tool, or gene tool for increasing the expression of GNAS gene.

The third embodiment of the present invention refers to a method for screening and/or producing compounds for use in a method for the treatment and/or prevention of HO which comprises assessing whether the candidate compound is able to reduce the amount or eliminate the cells comprising a GNAS loss-of-function mutation and lacking the GNAS wild-type allele, wherein if the compound is able to reduce the amount or eliminate said cells, it is an indication that the compound may be used for the treatment and/or prevention of HO.

The fourth embodiment of the present invention refers to the cell line of the invention ($\alpha$2) characterized by comprising a GNAS loss-of-function mutation and lacking the GNAS wild-type allele within its genetic material.

As explained above, this cell is sufficiently disclosed since the person skilled in the art could identify, with the above methods, whether the cell comprises a GNAS loss-of-function mutation and lacks the GNAS wild-type allele.

In any case, the cells of the invention ($\alpha$2) have been further characterized attending to differentially upregulated and downregulated miRNAs related to bone formation and tumour progression. Particularly, miRNA sequencing of $\alpha$1 and $\alpha$2 cells (GNAS single- and double-mutant cells, respectively) identified 2,751 miRNAs, of which 422 were differentially expressed (215 upregulated and 207 downregulated) with a threshold of $\pm$0.3 log2 fold change and a p-value cut-off of 0.05. To focus on those miRNAs with the greatest biological impact, these results were further filtered based on the number of transcripts per million (TPM), applying a cut-off of five times the median TPM for $\alpha$2 transcripts for miRNAs upregulated in $\alpha$2 cells and five times the median TPM for $\alpha$1 transcripts for miRNAs downregulated in $\alpha$2 cells, thus selecting the top 25% of miRNAs with the highest expression in $\alpha$2 and $\alpha$1 cells, respectively. Applying these criteria, 169 highly expressed miRNAs were upregulated in $\alpha$2 cells, while 169 highly expressed miRNAs in $\alpha$1 cells were found to be downregulated in $\alpha$2 cells.

The ten most upregulated miRNAs in $\alpha$2 cells (log2 fold change > 4, p < 0.0005, FDR < 0.008) included six miRNAs of the miR-302 cluster (miR-302a-3p, miR-302a-5p, miR-302b-3p, miR-302c-3p, miR-302c-5p, and miR-302d-3p). This cluster of miRNAs targets inhibitors of the BMP and TGF$\beta$ signalling pathways, thereby contributing to the formation of ectopic bone. The ten most downregulated miRNAs in $\alpha$2 cells (log2 fold change < -4, p < 0.0005, FDR < 0.008) comprised several miRNAs involved in tumour progression, such as miR-7156-3p and miR-135b-5p. MiR-7156-3p is negatively correlated with glioma grade, and its inhibition promotes glioma cell sternness, invasion, and growth. MiR-135b-5p reduces osteoblastoma growth and inhibits osteoblast growth and differentiation by targeting RUNX2.

The ten most upregulated and downregulated miRNAs in the cells of the invention ($\alpha$2) are summarised in the **Table 1** below.

**Table 1.** Top ten upregulated and downregulated miRNAs in α2 cells compared to α1 cells, as determined by microRNA sequencing.

| | #ID | log2FC | p-value | FDR |
|---|---|---|---|---|
| **Upregulated** | hsa-miR-302a-3p | 11.00 | 0.0000 | 0.0000 |
| | hsa-miR-302b-3p | 10.07 | 0.0000 | 0.0000 |
| | hsa-miR-302c-3p | 9.59 | 0.0000 | 0.0000 |
| | hsa-miR-3675-5p | 9.39 | 0.0000 | 0.0000 |
| | hsa-miR-302a-5p | 9.39 | 0.0000 | 0.0000 |
| | novel_miR_340 | 7.99 | 0.0000 | 0.0000 |
| | hsa-miR-302c-5p | 7.46 | 0.0000 | 0.0000 |
| | hsa-miR-302d-3p | 7.21 | 0.0000 | 0.0000 |
| | hsa-miR-372-3p | 5.69 | 0.0001 | 0.0023 |
| | hsa-miR-1269a | 4.34 | 0.0004 | 0.0074 |
| **Downregulated** | hsa-miR-6720-3p | -8.75 | 0.0000 | 0.0000 |
| | hsa-miR-598-3p | -8.04 | 0.0000 | 0.0000 |
| | hsa-miR-4664-3p | -7.78 | 0.0000 | 0.0000 |
| | hsa-miR-1267 | -6.81 | 0.0000 | 0.0000 |
| | hsa-miR-7156-5p | -6.69 | 0.0000 | 0.0001 |
| | hsa-miR-135b-5p | -5.94 | 0.0000 | 0.0000 |
| | novel_miR_526 | -5.35 | 0.0004 | 0.0070 |
| | novel_miR_160 | -4.21 | 0.0000 | 0.0000 |
| | novel_miR_9 | -4.15 | 0.0003 | 0.0047 |
| | hsa-miR-148a-5p | -4.06 | 0.0000 | 0.0000 |

Moreover, the cells of the invention (α2) have been further characterized attending to the proteome. The ten most upregulated proteins in α2 cells compared to α1 (FC > 4, p < 0.05) were eukaryotic translation initiation factor (EIF6), α-fetoprotein (AFP), copine-1 (CPNE1), poliovirus receptor (CD155), ATP-dependent RNA helicase DDX19B, ATP-dependent DNA helicase Q1 (RECQL), CTP synthase 1 (CTPS1), ATP-dependent 6-phosphofructokinase (PFKP), UDP-glucose 6-dehydrogenase (UGDH), and ATP-dependent RNA helicase DDX27. The ten most downregulated proteins (FC < 0.3, p < 0.05) were receptor expression-enhancing protein 5 (REEP5), cathepsin Z (CTSZ), protein S100-A4 (S100A4), protein NDRG1, BTB/POZ domain-containing protein KCTD12, galectin-3 (LGALS3), superkiller complex protein 3 (SKIC3), cathepsin B (CTSB), protein S100-A6 (S100A6), and heat shock protein beta-1 (HSPB 1).
In a preferred embodiment of the present invention, the mutation is NM_000516.7(GNAS):c.565_568del.
In a preferred embodiment of the present invention, the HO is a GNAS-related disease selected from the group comprising: POH, Albright hereditary dystrophy (AHO), pseudohypoparathyroidism (PHP), pseudopseudohypoparathyroidism (PPHP), and/or primary osteoma cutis.
In a preferred embodiment, the present invention refers to GNAS protein for use in a method for the treatment and/or prevention of HO, wherein the method comprises administering the exogenous GNAS protein to the patient. In a preferred embodiment, the method comprises administering the exogenous GNAS protein to the patient by means of lipid vesicles, polymers and/or by injection.
In a preferred embodiment, the gene editing tool is selected from the group comprising: CRISPR (Clustered Regularly Interspaced Short Palindromic Repeats), TALENs (Transcription Activator-Like Effector Nucleases), Zinc Finger Nucleases (ZFNs), Base Editors, Prime Editing, RNA Editing Tools, MegaTALs, or Oligonucleotide-Directed Mutagenesis (ODM).

In a preferred embodiment, the gene tool for increasing expression of GNAS is selected from the group comprising: CRISPR Activation (CRISPRa), Synthetic Transcription Factors, Viral Vectors, Plasmid Transfection, mRNA Transfection, Gene Amplification, Inducible Expression Systems, Epigenetic Modulation, Chemical Activators and/or Promoter Engineering, or self-amplifying mRNA.

The fifth embodiment of the present invention refers to a cell suspension or cell population that comprises the above defined cells or the secretome derived thereof.

Particularly, the secretome refers to a combination of molecules that are secreted by the cell. These molecules typically include proteins, lipids, nucleic acids, extracellular vesicles, and other bioactive substances that play critical roles in intercellular communication, signalling, and modulation of the extracellular matrix. The secretome is intrinsically linked to the genome of the cell, with its composition being a functional output of the genetic and epigenetic landscape, modulated by environmental cues.

In any case, the composition the α2 secretome has been analysed in the present invention. Proteomic analysis of the >100 kDa fraction of α1 and α2 secretomes identified α-2-macroglobulin (A2M) being the only one significantly up-regulated in α2 cells. Other proosteoblastogenic factors present in α2 secretome included apolipoprotein-A, COL1, periostin, vitronectin, and complement molecules C3 and C5.

The sixth embodiment of the present invention refers to a composition comprising the cell suspension or cell population of the invention, or the secretome derived thereof, for use in a method for promoting osteogenesis, for promoting chondrogenesis and/or for inhibiting adipogenesis. Alternatively, this embodiment also refers to a method for promoting osteogenesis, for promoting chondrogenesis and/or for inhibiting adipogenesis, which comprises the administration of a therapeutically effective amount of composition comprising the cell suspension or cell population of the invention, or the secretome derived thereof.

In a preferred embodiment, the composition comprising the cell suspension or cell population of the invention, or the secretome derived thereof, are used in a method for the treatment and/or prevention of a disease which may benefit from promoting the osteogenesis selected from: Osteoporosis, Osteogenesis Imperfecta (OI), Fracture Healing and Bone Defects, Paget's Disease of Bone, Osteomalacia and Rickets, Chronic Kidney Disease-Mineral and Bone Disorder (CKD-MBD), Osteonecrosis (Avascular Necrosis), Bone Loss from Prolonged Immobilization, Metabolic Bone Diseases, Bone Loss Due to Hormonal Changes and/or Bone Grafts and Orthopedic Surgeries; and/or for the treatment and/or prevention of diseases which may benefit from promoting chondrogenesis selected from: Osteoarthritis, rheumatoid Arthritis, traumatic cartilage injuries, intervertebral disc degeneration, chondrodysplasias, osteochondritis dissecans, avascular necrosis, temporomandibular joint disorders, congenital cartilage defects and/or post-surgical cartilage loss, and/or for the treatment and/or prevention of a disease which may benefit from inhibiting adipogenesis selected from obesity and metabolic syndrome, type 2 diabetes, cardiovascular diseases, non-alcoholic fatty liver disease (NAFLD), cancer or inflammatory diseases.

For the purpose of the present invention the following terms are defined:

- The term "comprising" means including, but not limited to, whatever follows the word "comprising". Thus, use of the term "comprising" indicates that the listed elements are required or mandatory, but that other elements are optional and may or may not be present.
- The term "consisting of" means including, and limited to, whatever follows the phrase "consisting of'. Thus, the phrase "consisting of" indicates that the listed elements are required or mandatory, and that no other elements may be present.
- By "therapeutically effective dose or amount" is intended an amount that, when administered as described herein, brings about a positive therapeutic response in a subject. The exact amount required will vary from subject to subject, depending on the species, age, and general condition of the subject, the severity of the condition being treated, mode of administration, and the like. An appropriate "effective" amount in any individual case may be determined by one of ordinary skill in the art using routine experimentation, based upon the information provided herein.

**Description of the figures**

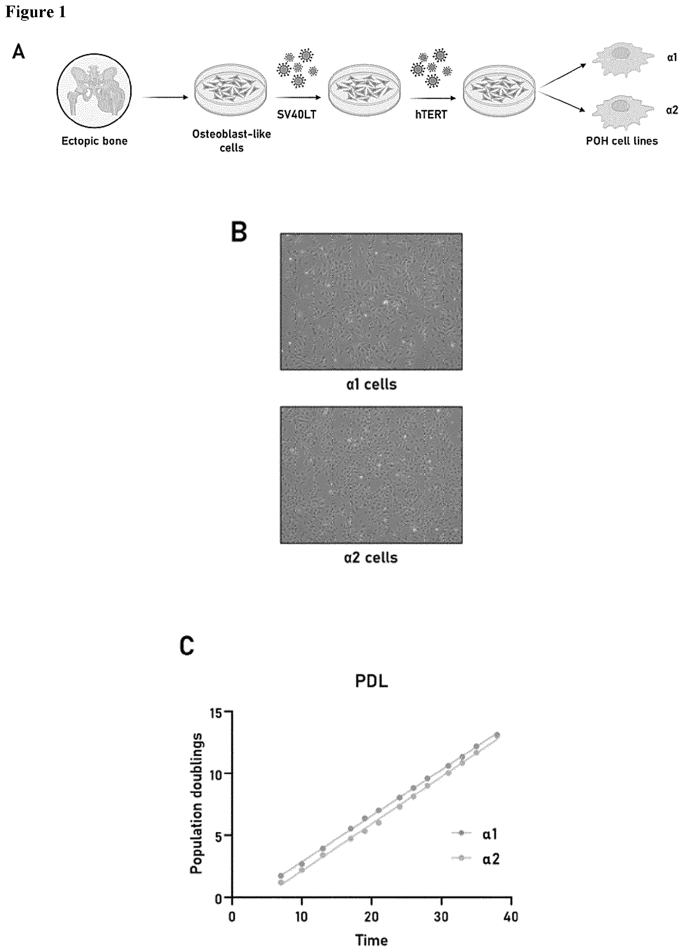

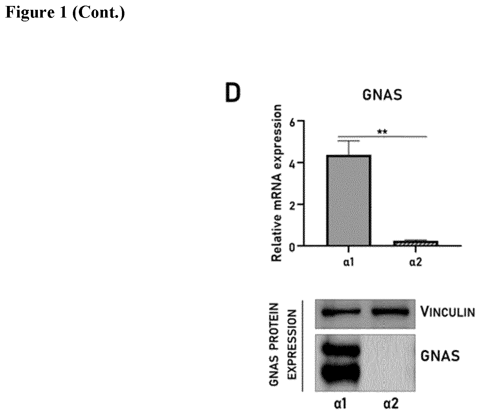

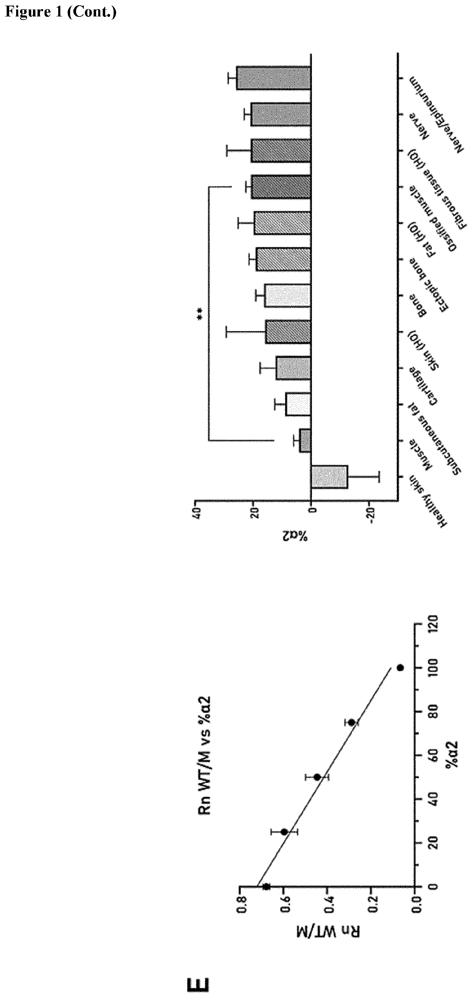

Figure 1. **(A)** Primary osteoblast-like cells were isolated from ectopic bone explants and transduced with simian virus 40 large T antigen (SV40LT). SV40LT-transduced cells were subsequently transduced with human telomerase reverse transcriptase (hTERT). Two clones, designated α1 and α2, were isolated from the immortalized cell population. **(B)** Phase contrast microscopic images of α1 and α2 cells. **(C)** Number of population doublings (PDs) accumulated by α1 and α2 cells against days in culture. PDs were calculated as (log Nf - log Ni)/log 2 (where Nf is the final cell population, Ni is the number of cells in the inoculum and log is the natural logarithm). PDL: population doublings level. **(D)** GNAS mRNA and protein expression in $\alpha 1$ and $\alpha 2$ cells; mRNA expression data was normalized to average. **(E)** Calibration curve obtained with the ratio of the Delta Rn values for the wild-type (WT) and mutated (M) alleles of different proportions of $\alpha 1$ and $\alpha 2$ cell mixtures against the percentage of $\alpha 2$ cells ($R$ squared = 0.9540). Percentage of $\alpha 2$-like cells (only M allele) in different tissues of the patient, as determined with the previous calibration curve. HO: tissue adjacent to ectopic bone. Bar graphs represent mean $\pm$ SEM. **, $p < 0.01$.

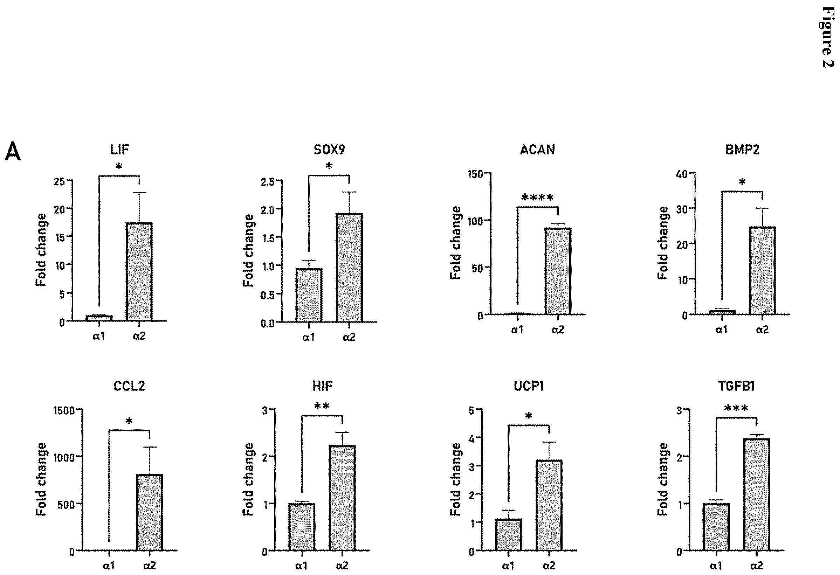

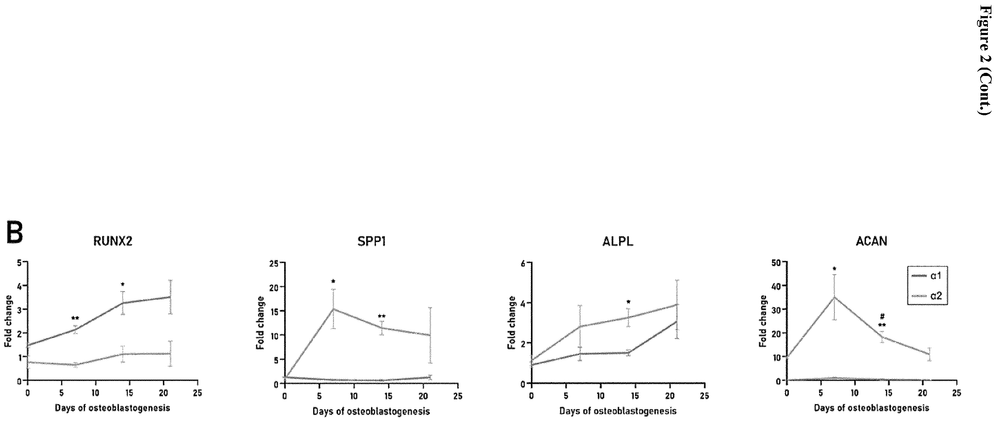

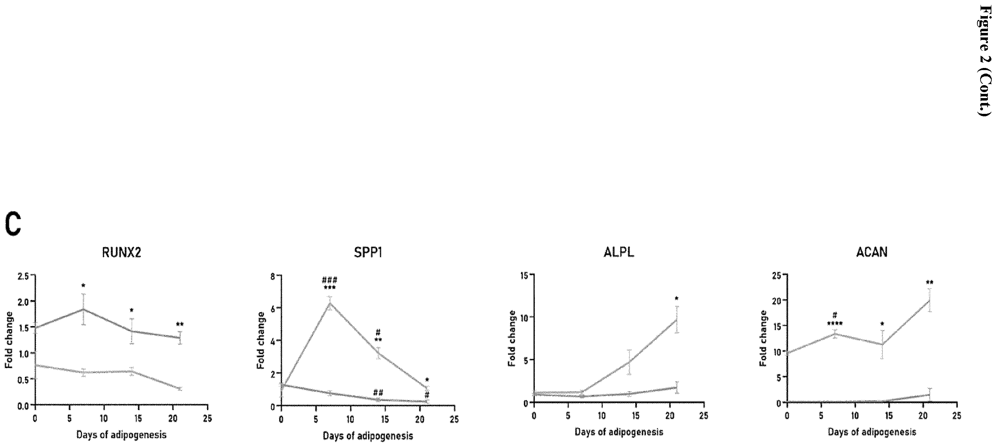

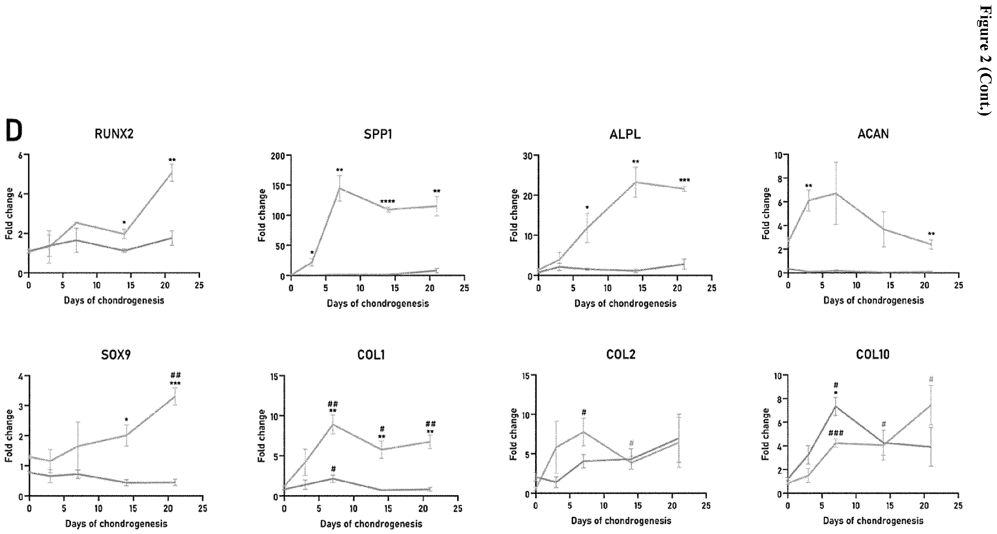

**Figure 2. (A)** Relative mRNA expression of leukemia inhibitory factor (LIF), SRY-Related HMG Box Gene 9 (SOX9), aggrecan (ACAN), bone morphogenetic protein 2 (BMP2), chemokine (C-C motif) ligand 2 (CCL), hypoxia-inducible factor (HIF), uncoupling protein 1 (UCP1), and transforming growth factor 1 (TGF$\beta$1) in $\alpha 1$ and $\alpha 2$ cells, in basal conditions. Data was normalized to clone $\alpha 1$. **(B)** Relative mRNA expression of Runt-related transcription factor 2 (RUNX2), osteopontin/secreted phosphoprotein-1 (SPP1), alkaline phosphatase (ALPL), and ACAN during 21 days of osteoblastogenic differentiation. **(C)** Relative mRNA expression of RUNX2, SPP1, ALPL, and ACAN during 21 days of adipogenic differentiation. **(D)** Relative mRNA expression of RUNX2, SPP1, ALPL, ACAN, SOX9, type I collagen (COL1), type II collagen (COL2), and type X collagen (COL10) during 21 days of chondrogenic differentiation. Data was normalized to average $\alpha 1$ and $\alpha 2$ expression at day 0. Graphs represent mean $\pm$ SEM. *, $p < 0.05$; **, $p < 0.01$; ***, $p < 0.001$; ****, $p < 0.0001$ (clone $\alpha 1$ *vs* clone $\alpha 2$, at the same day of differentiation). #, $p < 0.05$; ##, $p < 0.01$; ###, $p < 0.001$ (clone $\alpha 1/\alpha 2$ vs. day 0 of differentiation).

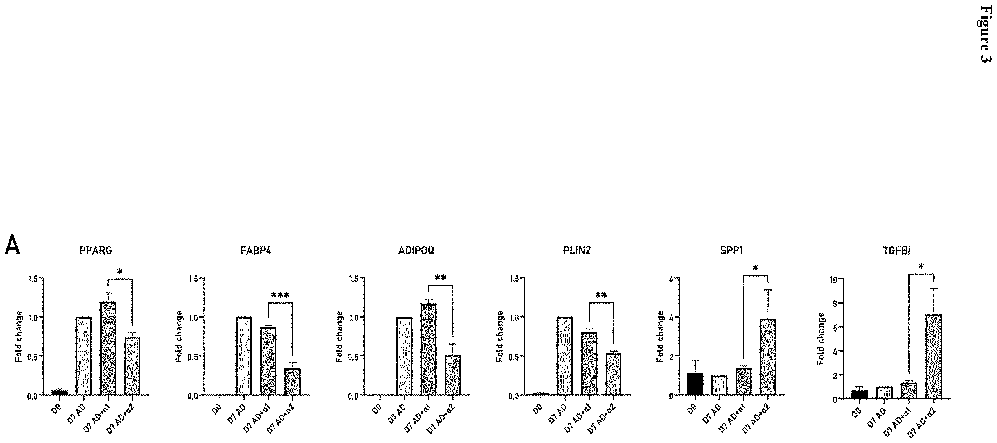

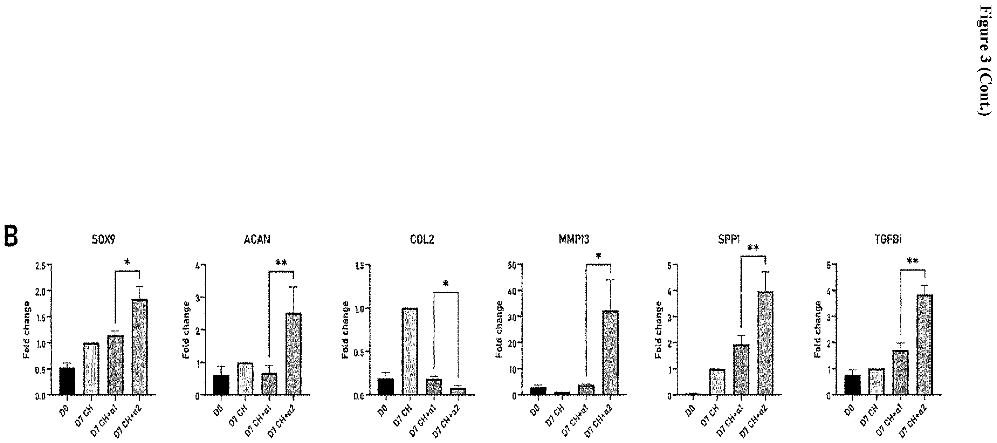

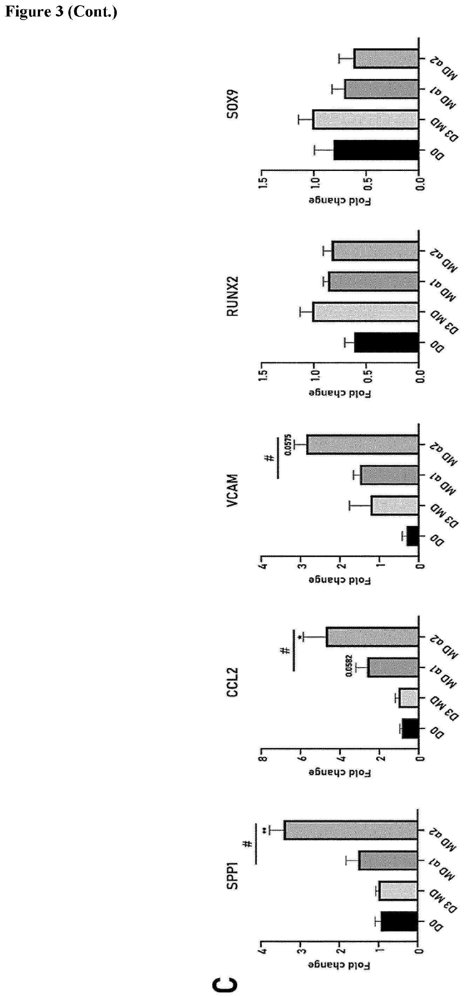

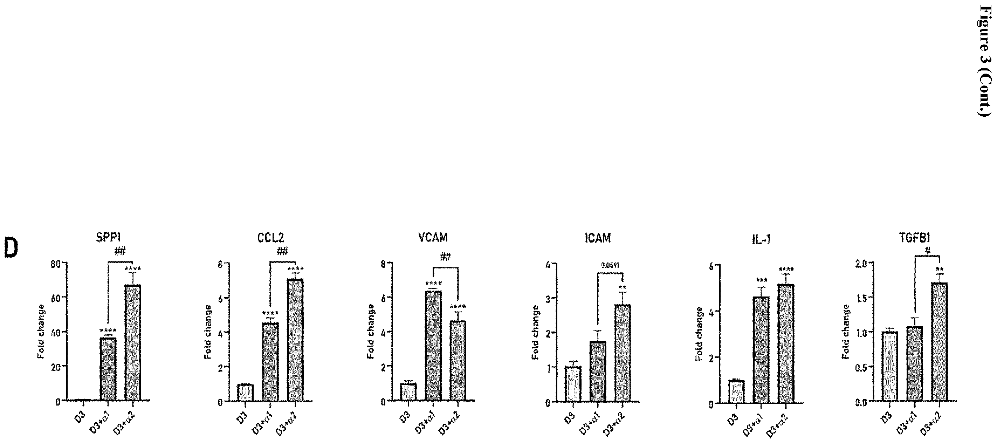

**Figure 3. (A)** Relative mRNA expression of peroxisome proliferator-activated receptor gamma (PPARG), fatty acid-binding protein 4 (FABP4), adiponectin (ADIPOQ), perilipin 2 (PLIN2), osteopontin/secreted phosphoprotein-1 (SPP1), and transforming growth factor $\beta$-induced (TGFBi) in C3H10T1/2 cells at day 0 and after 7 days of adipogenic differentiation with $\alpha 1$ and $\alpha 2$ cells secretomes; *, $p < 0.05$; **, $p < 0.01$; ***, $p < 0.001$. **(B)** Relative mRNA expression of SRY-Related HMG Box Gene 9 (SOX9), aggrecan (ACAN), type II collagen (COL2), collagenase 3 (MMP13), SPP1, and TGFBi in ATDC5 cells at day 0 and after 7 days of chondrogenic differentiation with $\alpha 1$ and $\alpha 2$ cells secretomes; *, $p < 0.05$; **, $p < 0.01$. **(C)** Relative mRNA expression of SPP1, chemokine (C-C motif) ligand 2 (CCL), vascular cell adhesion molecule (VCAM), Runt-related transcription factor 2 (RUNX2), and SOX9 in SaOS-2 cells at day 0 and after 3 days of osteoblastogenic differentiation with $\alpha 1$ and $\alpha 2$ cells secretome; *, $p < 0.05$; **, $p < 0.01$ (clone $\alpha 1/\alpha 2$ vs. day 3 of differentiation); #, $p < 0.05$ ($\alpha 1$ *vs.* $\alpha 2$). **(D)** Relative mRNA expression of SPP1, CCL2, VCAM, intercellular adhesion molecule (ICAM), interleukin-1 beta (IL-1$\beta$), and transforming growth factor $\beta$1 (TGF$\beta$1) in THP-1 mono-cytes after three days of culture and co-culture with $\alpha 1$ and $\alpha 2$ cells; **, $p < 0.01$; ***, $p < 0.001$; ****, $p < 0.0001$ (co-culture with $\alpha 1/\alpha 2$ *vs.* day 3 of culture); #, $p < 0.05$; ##, $p < 0.01$ ($\alpha 1$ *vs.* $\alpha 2$). Data was normalized to day 7 and day 3, respectively. Graphs represent mean $\pm$ SEM.

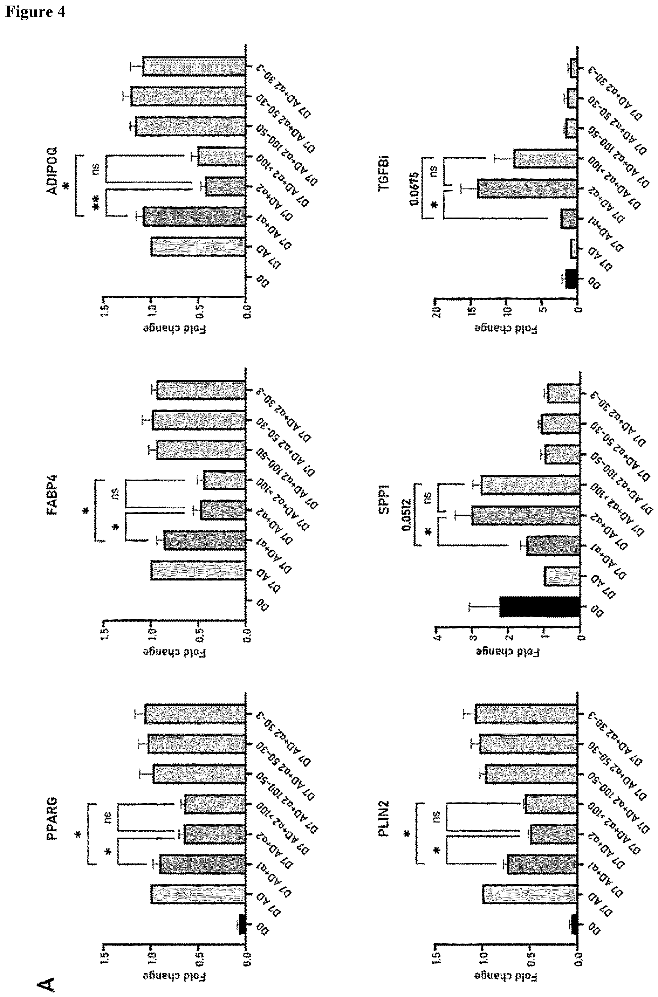

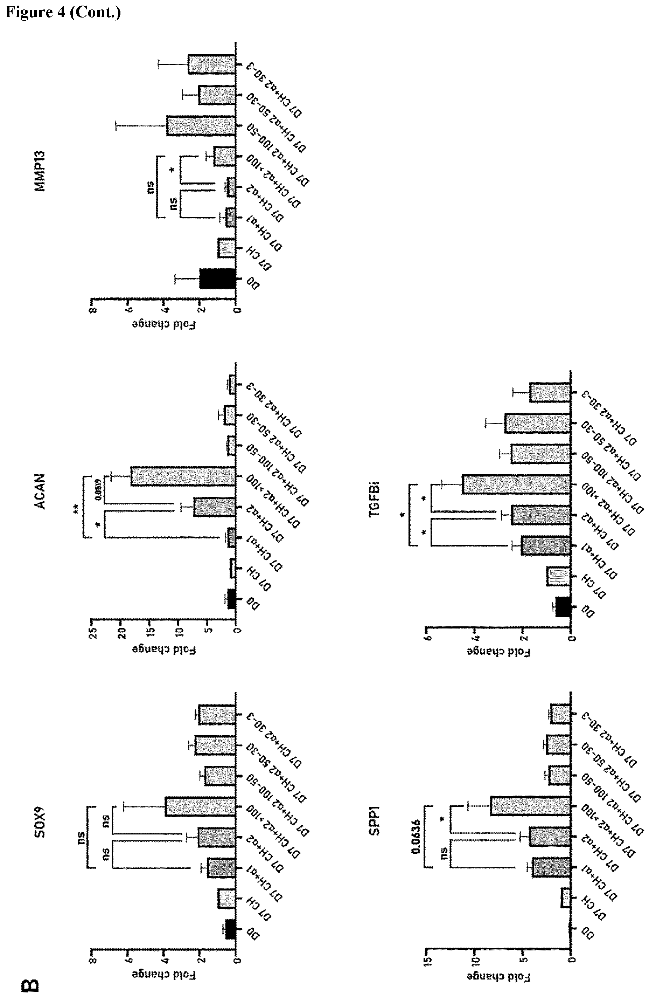

**Figure 4. (A)** Relative mRNA expression of peroxisome proliferator-activated receptor gamma (PPARG), fatty acid-binding protein 4 (FABP4), adiponectin (ADIPOQ), perilipin 2 (PLIN2), osteopontin/secreted phosphoprotein-1 (SPP1), and transforming growth factor $\beta$-induced (TGFBi) in C3H10T1/2 cells at day 0 and after 7 days of adipogenic differentiation with $\alpha 1$ and $\alpha 2$ cells secretomes and $\alpha 2$ secretome fractions. **(B)** Relative mRNA expression of SRY-Related HMG Box Gene 9 (SOX9), aggrecan (ACAN), type II collagen (COL2), collagenase 3 (MMP13), SPP1, and TGFBi in ATDC5 cells at day 0 and after 7 days of chondrogenic differentiation with $\alpha 1$ and $\alpha 2$ cells secretomes and $\alpha 2$ secretome fractions. Data was normalized to day 7. Graphs represent mean $\pm$ SEM; *, $p < 0.05$; **, $p < 0.01$.

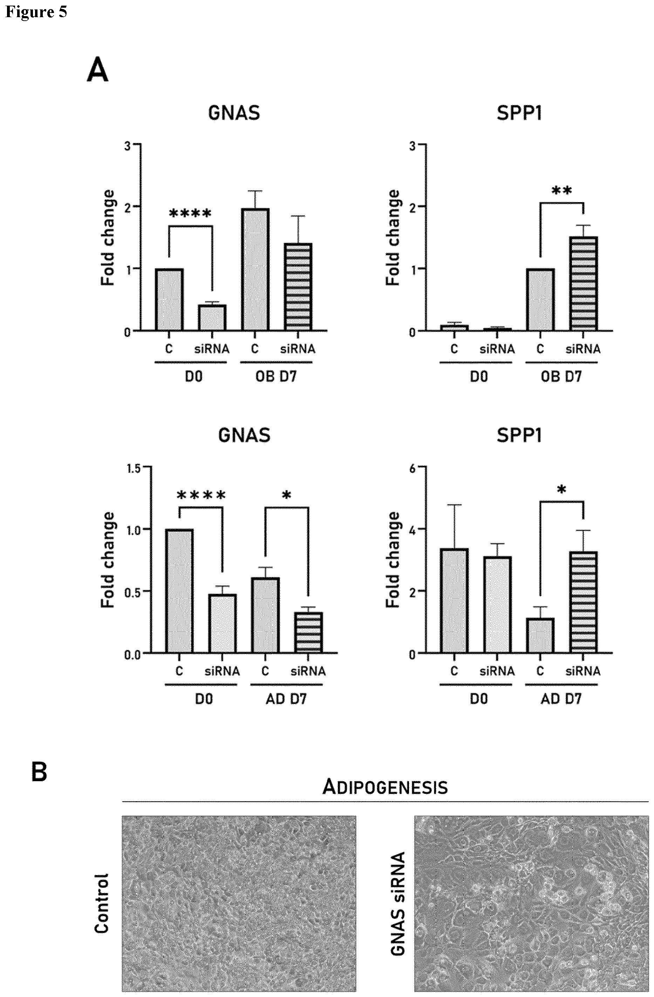

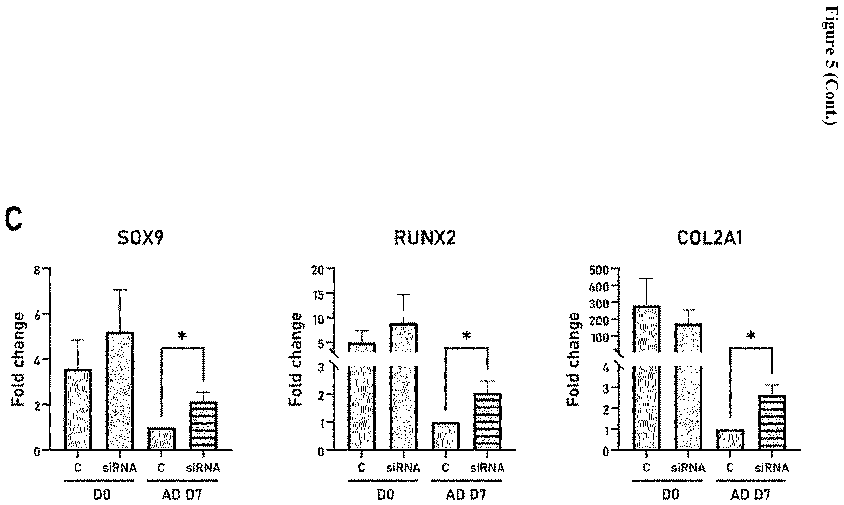

**Figure 5. (A)** Relative mRNA expression of GNAS and osteopontin/secreted phosphoprotein-1 (SPP1) in C3H10T1/2 after GNAS silencing (D0) and after 7 days of osteoblastogenic (OB) or adipogenic (AD) differentiation (D7). **(B)** Micrographs of C3H10T1/2 after 7 days of adipogenic differentiation following GNAS silencing, compared to control. **(C)** Relative mRNA expression of SRY-Related HMG Box Gene 9 (SOX9), Runt-related transcription factor 2 (RUNX2), and type II collagen (COL2) in C3H10T1/2 after GNAS silencing (D0) and after 7 days of adipogenic differentiation. Graphs represent mean $\pm$ SEM; *, $p < 0.05$; **, $p < 0.01$: ****, $p < 0.0001$.

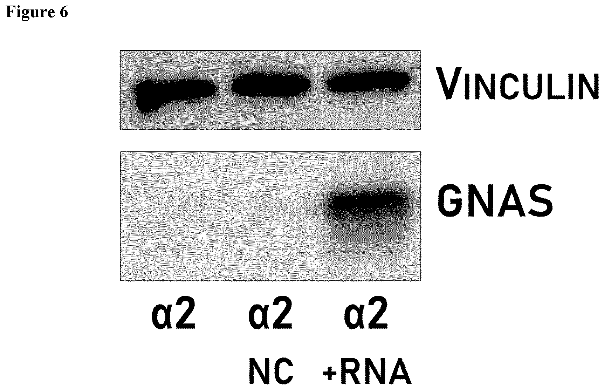

**Figure 6.** GNAS protein expression in $\alpha 2$ cells, $\alpha 2$ cells transfected with samRNA negative control (NC), and $\alpha 2$ cells transfected with GNAS samRNA (RNA).

## Detailed description of the invention

The present invention is illustrated by means of the Example set below without the intention of limiting its cope of protection.

## Example 1. MATERIAL AND METHODS

### Example 1.1. Cell isolation and culture

Primary osteoblast-like cells were isolated from ectopic bone explants obtained from a patient with POH who underwent surgical excision of ossified tissues. This patient was previously diagnosed with a heterozygous frameshift mutation in GNAS, consisting of a 4-base pair deletion in exon 7 (NM_000516.7(GNAS):c.565_568del). Bone explants were washed with PBS (Thermo Fisher Scientific, Waltham, MA, USA), placed in 100-mm adherent culture dishes (Costar Corning Incorporated, Corning, NY, USA), and cultured in basal medium, consisting of Dulbecco's modified Eagle's medium/Ham's F-12 (DMEM/F-12) supplemented with 10% foetal bovine serum (FBS), 4 mM L-glutamine, and 200 U/mL penicillin/streptomycin (all from Sigma-Aldrich, St. Louis, MO, USA). At confluence, osteoblast-like cells were subcultured with trypsin-EDTA solution (Sigma-Aldrich).

### Example 1.2. Cell immortalization

Primary osteoblast-like cells at the first passage were transduced by spinoculation using retrovirus produced by Phoenix Amphotropic cells (ATCC CRL-3213, φNX-A). Primary cells were transduced with simian virus 40 large T antigen (SV40LT), and SV40LT-transduced cells were subsequently transduced with human telomerase reverse transcriptase (hTERT) (**Figure 1A**). For retrovirus production, two plasmids were employed: pBABE-puro-SV40LT (Addgene plasmid #13970), deposited by Thomas Roberts, and pBABE-hygro-eGFP-hTERT (Addgene plasmid #28169), deposited by Kathleen Collins. Transduced cells were selected in 2.5 $\mu$g/mL puromycin (Sigma-Aldrich) or 50 $\mu$g/mL hygromycin (Thermo Fisher Scientific).

### Example 1.3. Clone isolation

Transduced cells were seeded at low density on 100 mm adherent culture dishes (100 cells/dish) and left to adhere for 18 hours. After verifying the clonal origin of each colony by microscopic observation with a Leica DMi1 inverted microscope (Leica Microsystems, Wetzlar, Germany), colonies were left to grow until reaching a suitable size for isolation. For clone isolation, 150 $\mu$L cloning cylinders (Sigma-Aldrich) were placed over each selected colony and cells were trypsinized and subcultured in adherent culture dishes. Two clones, designated $\alpha$1 and $\alpha$2, were selected for further characterization (**Figure 1A**).

### Example 1.4. Analysis of morphology and proliferation

The morphology of both clones was analysed by observation with a Leica DMi1 inverted microscope. The formula in Equation 1 was used to calculate the cumulative population doublings (PDs) of both clones. The number of PDs per day was calculated at each passage for 15 consecutive subcultures. Generation time was calculated for each cell line at each passage as the number of PDs per day. The proliferation rates of both clones were analysed by regression.

$$PD = \frac{\log Nf - \log Ni}{\log 2}$$

*Equation 1. Formula employed to calculate population doubling (PD) at each passage, where Nf is the final cell number, Ni is the initial cell number, and log is the natural logarithm.*

### Example 1.5. Cell differentiation

For $\alpha$1 and $\alpha$2 differentiation experiments, cells were plated in 24-well plates ($4.5 \times 10^4$ cells/well). Six hours after seeding, basal medium was replaced with differentiation medium. Osteoblastogenic medium consisted of DMEM high glucose supplemented with 10% FBS, 4 mM L-glutamine, 100 U/mL penicillin/streptomycin (DMEM10), 10 nM dexamethasone, 5 mM $\beta$-glycerol phosphate, and 50 $\mu$g/mL ascorbic acid-2-phosphate (all from Sigma-Aldrich). Adipogenic differentiation medium comprised DMEM10, 20 nM insulin-like growth factor 1 (IGF-1) (PeproTech Inc, Cranbury, NJ, USA), 2 $\mu$M rosiglitazone, 1 $\mu$M dexamethasone, 60 $\mu$M indomethacin, 0.5 mM 3-isobutyl-1-methylxanthine (IBMX), and 10 $\mu$g/mL insulin (all from Sigma-Aldrich). Adipogenic differentiation was induced by cycles consisting of 96 hours of adipogenic differentiation medium and 72 hours of maintenance medium (DMEM10 supplemented with 10 $\mu$g/mL insulin). Chondrogenic medium was DMEM high glucose supplemented with 2 mM L-glutamine, 100 U/mL penicillin/streptomycin, 10 ng/ml TGF-$\beta$3 (PeproTech Inc), 100 nM dexamethasone, 50 $\mu$g/mL ascorbic acid-2-phosphate, 40 $\mu$g/mL L-proline (Sigma-Aldrich), and 1X ITS+1 liquid media supplement (I2521, Sigma-Aldrich). Medium was renewed every 2-3 days.

In order to investigate the effect of $\alpha$1 and $\alpha$2 paracrine signals, cell differentiation and co-culture experiments with wild-type cell lines were carried out. To obtain conditioned medium from $\alpha1$ and $\alpha2$ clones, $1.5 \times 10^5$ cells were seeded per well in 6-well plates and cultured for 72 hours. For adipogenesis and chondrogenesis experiments, $\alpha1$ and $\alpha2$ were cultured in basal medium, and conditioned medium was filtered through 3-kDa Centricon filter units (Sigma-Aldrich), resuspended in differentiation medium, and filtered through a 0.22 $\mu$m-pore filter. For osteoblastogenesis experiments, $\alpha1$ and $\alpha2$ were cultured in SaOS2 differentiation medium, consisting of DMEM/F-12 supplemented with 10% FBS, 2 mM L-glutamine, 100 U/mL penicillin/streptomycin, and 50 $\mu$g/mL ascorbic acid-2-phosphate (Sigma-Aldrich). To further characterize the effect of $\alpha2$ secretome in adipogenesis and chondrogenesis, its protein content was separated by successive filtration using Centricon filter units with molecular mass cut-offs of 100, 50, 30 and 3 kDa.

For adipogenesis experiments, C3H10T1/2 cells were seeded in 24-well plates ($10^4$ cells/well) in DMEM10. Six hours after seeding, adipogenic differentiation was induced as described above. For chondrogenesis experiments, ATDC5 cells were seeded in 24-well plates ($5 \times 10^3$ cells/well) in growth medium, consisting of DMEM/F12 supplemented with 5% FBS, 4 mM L-glutamine, 200 U/mL penicillin/streptomycin, 5 $\mu$g/mL transferrin, and 30 nM sodium selenite. One day after seeding, growth medium was replaced by chondrogenic medium, consisting of growth medium supplemented with 0.25 UI/mL insulin. Medium was renewed every 2-3 days. After 7 days of differentiation, cells were lysed to obtain RNA.

For osteoblastogenesis experiments, SaOS2 cells were seeded in 6-well plates ($1.2 \times 10^5$ cells/well) in growth medium, consisting of DMEM/F12 supplemented with 10% FBS, 2 mM L-glutamine, and 100 U/mL penicillin/streptomycin. One day after seeding, culture medium was replaced by SaOS2 differentiation medium. Cells were lysed to obtain RNA after 3 days of culture.

### Example 1.6. Co-culture experiments

For co-culture with THP-1 monocytes, $10^4$ cells of $\alpha1$ and $\alpha2$ clones were seeded in 0.4 $\mu$m-pore cell culture inserts (Costar Corning Incorporated), and $1.5 \times 10^5$ cells were placed per well in 24-well plates (both in basal medium). One day after seeding, the inserts containing $\alpha1$ and $\alpha2$ cells were placed over THP-1 wells. After 72 hours of co-culture, THP-1 cells were pelleted and lysed to obtain RNA.

### Example 1.7. GNAS silencing

C3H10T1/2 cells were seeded on 24-well plates at a density of $10^4$ cells per well. The following day, culture medium was changed for DMEM/F12 supplemented with 2% FBS. After 1-hour incubation, GNAS silencing was performed using the TriFECTa DsiRNA Kit and the siLentFect™ Lipid Reagent for RNAi (Integrated DNA Technologies, Coralville, IO, USA) in Opti-MEM (Gibco, Thermo Fisher Scientific). A negative control DsiRNA was included to confirm that the transfection process did not affect normal cell function. After 6-hour incubation, the medium was discarded, and the cells were subjected to adipogenic and osteoblastic differentiation as previously described. Cells were lysed for RNA extraction after 7 days of differentiation.

### Example 1.8. GNAS self-amplifying mRNA transfection

Self-amplifying mRNA (samRNA) coding for GNAS (UniProt accession number P63092) was synthesized by GenScript (Piscataway, NJ, USA). One day before transfection, $\alpha2$ cells were seeded on 24-well plates at a density of $80^4$ cells per well. Lipofectamine MessengerMAX (Thermo Fisher Scientific) and 0.5 ng of GNAS samRNA were mixed in Opti-MEM and added to $\alpha2$ cells. Cells were incubated with the samRNA-lipid complex for 24 hours. Negative control wells were subjected to the same procedure without the GNAS samRNA.

### Example 1.9. TaqMan SNP Genotyping Assay

Tissues obtained from surgical leavings were immersed in liquid nitrogen and pulverized using a Cellcrusher (Schull, Ireland). Cytiva triplePrep Kit (Thermo Fisher Scientific) was employed to obtain genomic DNA from 20 mg of pulverized tissue or $10^6$ cultured cells. DNA quantity was determined using a NanoDrop 2000 Spectrophotometer and 5 ng were mixed with Maxima Probe/ROX qPCR Master Mix and TaqMan primers and probes specifically designed for the mutation site (NM_000516.7(GNAS):c.565_568del) (all from Thermo Fisher Scientific). TaqMan SNP Genotyping Assay was carried out in a QuantStudio 3 Real-Time PCR system (Thermo Fisher Scientific). For each sample, the ratio between the Delta Rn values for the wild-type allele (WT) and the mutated allele (M) were calculated. In order to determine the proportion of $\alpha1/\alpha2$ mosaicism in each tissue, a calibration curve was made by mixing $\alpha1$ and $\alpha2$ cells in different proportions.

## Example 1.10. Gene expression analysis

Cells were lysed using TRI Reagent (Sigma-Aldrich) and RNA was purified using E.Z.N.A. total RNA kit I (Omega Bio-Tek, Norcross, GA, USA), according to the manufacturer's instructions. RNA was treated with RNase-free Dnase I (Lucigen, Middleton, WI, USA) and retro-transcribed employing the High-Capacity RNA-To-cDNA (Thermo Fisher Scientific). Gene expression levels were assessed by real-time PCR using iTaq Universal SYBR Green Supermix (BioRad, Hercules, CA, USA) in combination with specific primers in a QuantStudio 3 Real-Time PCR system. Data analysis was performed using the QuantStudio 3/5 Real-Time PCR Software (Thermo Fisher Scientific). Relative quantification was obtained by the $2^{-\Delta\Delta Ct}$ method with HPRT as the reference gene.

## Example 1.11. Western blotting

Proteins were isolated using RIPA buffer (Sigma-Aldrich) with Halt Protease and Phosphatase Inhibitor Cocktail (Thermo Fisher Scientific) and quantified using BSA Pierce Bradford assay (Thermo Fisher Scientific). Protein samples (25 µg) were loaded onto a 10% sodium dodecyl sulfate polyacrylamide gel. After electrophoresis (SDS-PAGE), proteins were transferred to a polyvinylidene difluoride (PVDF) membrane and incubated at 4°C overnight with the primary antibodies: rabbit monoclonal anti-GNAS (ab283266; 1:500) and mouse monoclonal anti-vinculin (ab13007; 1:1000), both from Abcam (Cambridge, UK). Incubation with the secondary antibodies goat anti-rabbit (1:5,000) and goat anti-mouse (1:10,000), both from Jackson ImmunoResearch (West Grove, PA, USA), was performed for 1 hour at room temperature. Target proteins were visualized on a ChemiDoc MP Imaging System (BioRad) employing Immobilon Chemiluminescent HRP Substrate (ECL) (Millipore, Sigma-Aldrich).

## Example 1.12. RNA sequencing

RNA-seq analysis was performed by the Transcriptomics Service of IDIS using the Illumina NextSeq 2000 platform. KEGG pathway enrichment analysis was performed and rendered using Pathview.

## Example 1.13. Proteomic analysis

Proteomic analysis was performed by the Proteomics Service of IDIS. Micro liquid chromatography-mass spectrometry (micro-LC-MS/MS) was performed using a hybrid quadrupole TripleTOF 6600 (SCIEX, Framingham, MA, USA). Proteomes were identified by the qualitative shot-gun data-dependent acquisition (DDA) method, and protein levels were measured by the quantitative sequential window acquisition of all theoretical mass spectra (SWATH) method. A 5% false discovery rate (FDR) and p-value ≤ 0.05 were used to filter the dataset. Enrichment analysis was performed using the ARCHS4 Tissues database in Enrichr to identify tissue-specific gene expression patterns.

## Example 2. RESULTS

## Example 2.1. Loss of GNAS wild-type allele in ectopic bone and surrounding tissues

Clone α1 displayed a fibroblast-like cell morphology characteristic of mesenchymal stromal cells and pre-osteoblasts, while clone α2 was more polygonal-shaped (**Figure 1B**). For both clones, a continuous growth ratio was evidenced after regression analysis (R > 0.99 and p-value < 0.0001) (**Figure 1C**). Mean generation time of was 2.74 ± 0.32 days for clone α1 and 2.65 ± 0.36 days for clone α2. There were no significant differences among clones regarding proliferation rate (p-value = 0.4768). The expression of transgenes SV40LT and hTERT was evidenced in both clones. Interestingly, clone α2 showed an almost twenty-fold reduction in GNAS gene expression in comparison with clone α1, and no detectable GNAS protein expression (**Figure 1D**). TaqMan SNP Genotyping Assay revealed that clone α1 was heterozygous for the mutation (NM_000516.7(GNAS):c.565_568del), while only the mutated allele was detected in clone α2. Employing a calibration curve obtained from the Delta Rn values for the wild-type (WT) and mutated (M) alleles of different proportions of α1 and α2 cell mixtures, the percentage of α2-like cells (only M allele, i.e. double-mutant) for different tissues were determined. The proportion of double-mutant, α2-like cells was higher in ossified tissues, such as muscle, and tissues adjacent to ectopic bone, such as fat and fibrous tissue. The highest percentage of α2-like cells was found in nervous tissues. Surprisingly, certain tissues, including healthy skin, had a Delta Rn ratio higher than α1 cells, suggesting the presence of cells with only WT allele (**Figure 1E**).

## Example 2.2. Biallelic loss of GNAS increases the expression of bone and cartilage-related genes

In addition to differing expression levels of GNAS, clones α1 and α2 showed differential expression of genes associated with the process of ectopic bone formation. The expression of leukaemia inhibitory factor (LIF), an inductor of ossification, was almost twenty times higher in $\alpha$2 cells. The expression of SRY-Related HMG Box Gene 9 (SOX9), a key transcription factor for chondrogenesis, and aggrecan (ACAN), one of the main components of cartilage extracellular matrix, was also higher in $\alpha$2 cells. The same trend was followed by BMP2, an activator of osteogenic genes; chemokine (C-C motif) ligand 2 (CCL2), a monocyte chemoattractant protein; hypoxia-inducible factor (HIF), an inductor of angiogenesis and cartilage differentiation; uncoupling protein 1 (UCP1), expressed by ectopic bone-associated brown adipocyte-like cells; and TGF$\beta$1, an inductor of ectopic bone formation (**Figure 2A**).

Clone $\alpha$1 showed higher expression of the bone-related transcription factor Runt-related transcription factor 2 (RUNX2) under osteoblastogenic and adipogenic *stimuli* (**Figure 2B**-C), while clone $\alpha$2 showed a significant upregulation of its expression under chondrogenic *stimuli* (**Figure 2D**). Interestingly, even though there was no difference in SPP1 expression at basal conditions, clone $\alpha$2 showed a notable upregulation under all the differentiation *stimuli,* especially during chondrogenesis (**Figure 2B-D**). Strikingly, SPP1 was downregulated in clone $\alpha$1 but upregulated in clone $\alpha$2 during adipogenesis (**Figure 2C**). Alkaline phosphatase (ALPL) expression was also higher in $\alpha$2 cells under all three conditions, with the most notable differences found upon chondrogenic induction (**Figure 2B-D**). The expression of the cartilage marker aggrecan (ACAN) was also significantly higher in clone $\alpha$2 in all three conditions, and its expression was induced in clone $\alpha$2 under all three *stimuli* (**Figure 2B-D**). Moreover, during chondrogenesis, SOX9 expression was induced in clone $\alpha$2, but not in clone $\alpha$1. Unexpectedly, type I collagen (COL1) was also upregulated in clone $\alpha$2 during chondrogenesis. Type II collagen (COL2) was upregultaed only in clone $\alpha$2 during the same process, while type X collagen was upregulated in both clones (COL10) (**Figure 2D**).

**Example 2.3. GNAS double-mutant cells inhibit adipogenesis, induce osteochondrogenesis and promote inflammation through paracrine signalling**

Through cell differentiation experiments in wild type cell lines, the secretome of $\alpha$2 cells was demonstrated to have adipogenesis-inhibiting and chondrogenesis-inducing activity. After 7 days of adipogenic differentiation, all the adipogenesis-related genes studied (peroxisome proliferator-activated receptor $\gamma$ (PPARG), fatty acid-binding protein 4 (FABP4), adiponectin (ADIPOQ), and perilipin 2 (PLIN2)) were upregulated in C3H10T1/2 cells; however, $\alpha$2 cells secretome reduced the induction of all four genes, unlike $\alpha$1 cells secretome (**Figure 3A**). On ATDC5 cells subjected to 7-day chondrogenic induction, $\alpha$2 cells secretome induced the expression of SOX9 and ACAN but reduced the expression of COL2. The expression of collagenase 3 (MMP13) was also significantly induced (**Figure 3B**). In addition, $\alpha$2 cells secretome significantly induced the expression of SSP1 and transforming growth factor $\beta$-induced (TGFBi), two factors closely related to HO pathogenesis, in both adipogenically- and chondrogenically-induced cells (**Figure 3A-B**).

On the osteoblastic cell line SaOS-2, $\alpha$2 cells secretome also induced the expression of SPP1, as well as that of the chemotactic factors CCL2 and vascular cell adhesion molecule (VCAM), while the expression of the transcription factors RUNX2 and SOX9 remained unchanged (**Figure 3C**). In co-culture experiments, $\alpha$2 cells induced the expression of SPP1, CCL2, VCAM, ICAM, IL-1$\beta$, and TGF$\beta$1 in THP-1 monocytes. While $\alpha$1 cells also induced the expression of some of these genes, all of them related to the pathogenic ossification process, the upregulation of the key factors SPP1, CCL2, and TGF$\beta$1 in monocytes cocultured with $\alpha$2 cells were significantly higher (**Figure 3D**). Overall, these results demonstrate the ability of $\alpha$2 cell secretome to induce the expression of SPP1 and activate TGF$\beta$1 signalling, both of which are key factors in ectopic bone formation, across a broad range of cell types.

In order to identify which protein(s) were responsible for the $\alpha$2 paracrine effects, the same experiments were performed using the secretome of $\alpha$2 cells divided into fractions of decreasing molecular weights. Interestingly, only the fraction containing proteins larger than 100 kDa showed the same adipogenesis-inhibiting activity as the whole secretome (**Figure 4A**). Furthermore, this protein fraction exhibited a chondrogenesis-inducing effect similar to that of the whole secretome and retained the ability to induce the expression of SPP1 and TGFBi under both adipogenic and chondrogenic stimulation (**Figure 4A-B**).

**Example 2.4. Silencing of GNAS inhibits adipogenesis and induces osteochondrogenesis**

GNAS silencing in the mesenchymal progenitor cell line C3H10T1/2 resulted in enhanced SPP1 expression after 7 days of either osteoblastogenic or adipogenic differentiation, even though GNAS silencing was not sustained during osteoblastogenesis (**Figure 5A**). During adipogenesis, GNAS silencing impaired the formation of lipid droplets (**Figure 5B**) and increased the expression of the cartilage- and bone-related transcription factors SOX9 and RUNX2, as well as the main collagen of growth plate cartilage, COL2 (**Figure 5C**).

**Example 2.5. GNAS single- and double-mutant cells show widespread proteomic divergence**

A total of 1,185 proteins were detected in $\alpha$1 and $\alpha$2 clones by SWATH mass spectrometry, out of which 685 (58%) were differentially expressed; 336 proteins were upregulated in α2 cells, while the other 349 were downregulated. The ten most upregulated proteins in α2 cells compared to α1 (FC > 4, p < 0.05) were eukaryotic translation initiation factor (EIF6), α-fetoprotein (AFP), copine-1 (CPNE1), poliovirus receptor (CD155), ATP-dependent RNA helicase DDX19B, ATP-dependent DNA helicase Q1 (RECQL), CTP synthase 1 (CTPS1), ATP-dependent 6-phosphofructokinase (PFKP), UDP-glucose 6-dehydrogenase (UGDH), and ATP-dependent RNA helicase DDX27. The ten most downregulated proteins (FC < 0.3, p < 0.05) were receptor expression-enhancing protein 5 (REEP5), cathepsin Z (CTSZ), protein S100-A4 (S100A4), protein NDRG1, BTB/POZ domain-containing protein KCTD12, galectin-3 (LGALS3), superkiller complex protein 3 (SKIC3), cathepsin B (CTSB), protein S100-A6 (S100A6), and heat shock protein beta-1 (HSPB1). In contrast, DDA proteomic analysis of the >100 kDa fraction of α1 and α2 secretomes identified only 42 proteins, with α-2-macroglobulin (A2M) being the only one significantly up-regulated in α2 cells. Other proosteoblastogenic factors present in α2 secretome included apolipoprotein-A, COL1, periostin, vitronectin, and complement molecules C3 and C5.

### Example 2.6. Transcriptomic analysis of double-mutant cells reveals enrichment of genes related to bone formation and inflammation

RNA-seq analysis of α1 and α2 cells identified 5,189 differentially expressed genes (2,618 upregulated and 2,571 downregulated), with a threshold of ±0.3 log2 fold change and a p-value cut-off of 0.05. Consistent with our previous findings and the phenotypic differences observed between the two cell lines, genes related to bone formation (BMP2, LIF), cartilage (ACAN), and chemotaxis (CCL2) were upregulated in α2 cells. Other upregulated genes included matrix degrading enzymes related to bone metastasis (MMP-1), endochondral ossification (MMP-10), and adipogenesis inhibition (ADAMTS18), as well as chemokines CCL7, CXCL9, CXCL5, and CXCL11. Interestingly, one of the most upregulated genes was the transcription factor SOX11, whose expression is significantly induced in adult neurons in response to injury. The transcription factor RUNX3, which is activated by TGFβ and BMP signalling and promotes chondrocyte maturation during osteochondral ossification, was also highly upregulated. In contrast, transcription factors associated with adipogenesis, such as PPARγ, C/EBPα, C/EBPδ, and KLF5, were downregulated in α2 cells. As expected, GNAS was also markedly downregulated. Similarly, KEGG pathway enrichment analysis revealed significant downregulation of cAMP signalling and adipocyte lipolysis, while inflammatory pathways such as chemokines, TLRs, TNF and IL-17 signalling were upregulated. Enrichment analysis using the ARCHS4 Tissues database showed that the top enriched term for α2 cells was "astrocyte" (adjusted p-value = $5.328 \times 10^{-7}$, combined score = 27.26), while for α1 cells, it was "fibroblast" (adjusted p-value = $3.089 \times 10^{-58}$, combined score = 347.77).

### Example 2.7. Self-amplifying mRNA transfection restores the expression of GNAS

The transfection with GNAS samRNA restored GNAS expression in α2, double-mutant cells, after 24 hours of treatment (**Figure 6**).

### Claims

1. *In vitro* method for the diagnosis and/or prognosis of heterotopic ossification which comprises assessing the presence of cells **characterized in that** they comprise a GNAS loss-of-function mutation and lack the GNAS wild-type allele, in a biological sample obtained from the subject, wherein the presence of said cells is indicative that the subject may be suffering from heterotopic ossification.

2. GNAS protein, gene editing tool, or gene tool for increasing the expression of GNAS gene, for use in a method for the treatment and/or prevention of heterotopic ossification wherein the method comprises reducing the amount or eliminating cells comprising a GNAS loss-of-function mutation and lacking the GNAS wild-type allele.

3. Method for screening and/or producing compounds for use in a method for the treatment and/or prevention of heterotopic ossification which comprises assessing whether the candidate compound is able to reduce the amount or eliminate the cells comprising a GNAS loss-of-function mutation and lacking the GNAS wild-type allele, wherein if the compound is able to reduce the amount or eliminate said cells, it is an indication that the compound may be used for the treatment and/or prevention of heterotopic ossification.

4. Cell line **characterized by** comprising a GNAS loss-of-function mutation and lacking the GNAS wild-type allele within its genetic material, or the secretome derived thereof.

5. *In vitro* method for the diagnosis and/or prognosis, according to claim 1, GNAS protein, gene editing tool, or gene tool for increasing the expression of GNAS gene, for use, according to claim 2, method for screening according to claim 3, or cell line according to claim 4, wherein the mutation is NM_000516.7(GNAS):c.565_568del.

6.  *In vitro* method for the diagnosis and/or prognosis, according to claims 1 or 5, GNAS protein, gene editing tool, or gene tool for increasing the expression of GNAS gene, for use, according to claims 2 or 5, or method for screening according to claims 3 or 5, wherein heterotopic ossification is a GNAS-related disease selected from the group comprising: Progressive Osseous Heteroplasia (POH), Albright hereditary dystrophy (AHO), pseudohypoparathyroidism (PHP), pseudopseudohypoparathyroidism (PPHP), and/or primary osteoma cutis.

7.  GNAS protein for use, according to claims 2, 5 or 6, in a method for the treatment and/or prevention of heterotopic ossification, wherein the method comprises administering the exogenous GNAS protein to the patient.

8.  GNAS protein for use, according to claims 2, 5, 6 or 7, wherein the method comprises administering the exogenous GNAS protein to the patient by means of lipid vesicles, polymers and/or by injection.

9.  Gene editing tool for use, according to claims 2, 5 or 6, selected from the group comprising: CRISPR (Clustered Regularly Interspaced Short Palindromic Repeats), TALENs (Transcription Activator-Like Effector Nucleases), Zinc Finger Nucleases (ZFNs), Base Editors, Prime Editing, RNA Editing Tools, MegaTALs, or Oligonucleotide-Directed Mutagenesis (ODM).

10. Gene tool for increasing expression of GNAS for use, according to claims 2, 5 or 6, selected from the group comprising: CRISPR Activation (CRISPRa), Synthetic Transcription Factors, Viral Vectors, Plasmid Transfection, mRNA Transfection, Gene Amplification, Inducible Expression Systems, Epigenetic Modulation, Chemical Activators and/or Promoter Engineering, or self-amplifying mRNA.

11. Cell suspension or cell population comprising the cells according to any of the claims 4 or 5, or the secretome derived thereof.

12. A composition comprising the cell suspension or cell population of claim 11, or the secretome derived thereof, for use in a method for promoting osteogenesis, for promoting chondrogenesis and/or for inhibiting adipogenesis.

13. Composition for use, according to claim 12, in a method for the treatment and/or prevention of a disease which may benefit from promoting the osteogenesis selected from: Osteoporosis, Osteogenesis Imperfecta (OI), Fracture Healing and Bone Defects, Paget's Disease of Bone, Osteomalacia and Rickets, Chronic Kidney Disease-Mineral and Bone Disorder (CKD-MBD), Osteonecrosis (Avascular Necrosis), Bone Loss from Prolonged Immobilization, Metabolic Bone Diseases, Bone Loss Due to Hormonal Changes and/or Bone Grafts and Orthopedic Surgeries; and/or for the treatment and/or prevention of diseases which may benefit from promoting chondrogenesis selected from: Osteoarthritis, rheumatoid Arthritis, traumatic cartilage injuries, intervertebral disc degeneration, chondrodysplasias, osteochondritis dissecans, avascular necrosis, temporomandibular joint disorders, congenital cartilage defects and/or post-surgical cartilage loss, and/or for the treatment and/or prevention of a disease which may benefit from inhibiting adipogenesis selected from obesity and metabolic syndrome, type 2 diabetes, cardiovascular diseases, non-alcoholic fatty liver disease (NAFLD), cancer or inflammatory diseases.

**Figure 1**

A

Ectopic bone → Osteoblast-like cells → SV40LT → hTERT → POH cell lines (α1, α2)

B

α1 cells

α2 cells

C

PDL

**Figure 1 (Cont.)**

**Figure 1 (Cont.)**

**Figure 2**

**Figure 2 (Cont.)**

**Figure 2 (Cont.)**

Figure 2 (Cont.)

Figure 3

**Figure 3 (Cont.)**

**Figure 3 (Cont.)**

Figure 3 (Cont.)

Figure 4

Figure 4 (Cont.)

**Figure 5**

**Figure 5 (Cont.)**

Figure 6

| | Europäisches Patentamt European Patent Office Office européen des brevets | **EUROPEAN SEARCH REPORT** | **Application Number** EP 25 38 2052 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HALDEMAN-ENGLERT ET AL: "Disorders of GNAS Inactivation - GeneReviews - NCBI Bookshelf", , 26 October 2017 (2017-10-26), XP093289521, Retrieved from the Internet: URL:https://www.ncbi.nlm.nih.gov/books/NBK459117/ [retrieved on 2025-06-25] * page 1, line 1 - line 30 * * page 5, line 3 - line 11 * * page 10 - page 11; table 2 * * page 21; table 5 * ----- | 1-13 | INV. C12Q1/6883 C12N5/077 |
| X | TURAN SERAP ET AL: "GNASSpectrum of Disorders", CURRENT OSTEOPOROSIS REPORTS, SPRINGER US, NEW YORK, [Online] vol. 13, no. 3, 8 April 2015 (2015-04-08), pages 146-158, XP035497130, ISSN: 1544-1873, DOI: 10.1007/S11914-015-0268-X [retrieved on 2025-06-26] * abstract * * page 148; figure 2 * * page 149, right-hand column, paragraph three - paragraph four * ----- -/-- | 1-13 | |

| | | | TECHNICAL FIELDS SEARCHED (IPC) |
|---|---|---|---|
| | | | C12Q C12N |

The present search report has been drawn up for all claims

| Place of search | Date of completion of the search | Examiner |
|---|---|---|
| Munich | 2 July 2025 | Bradbrook, Derek |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 25 38 2052

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PIGNOLO ET AL: "Progressive osseous heteroplasia: diagnosis, treatment, and prognosis - PMC", THE APPLICATION OF CLINICAL GENETICS, [Online] 1 January 2015 (2015-01-01), page 37, XP093289512, ISSN: 1178-704X, DOI: 10.2147/TACG.S51064 Retrieved from the Internet: URL:https://pmc.ncbi.nlm.nih.gov/articles/ PMC4321643/#sec8> [retrieved on 2025-06-25] * abstract * * page 41; table 1 * * page 44; figure 5 * * page 45, left-hand column, paragraph three - paragraph four * ----- | 1-13 | |
| X | MANTOVANI GIOVANNA ET AL: "Diagnosis and management of pseudohypoparathyroidism and related disorders: first international Consensus Statement", NATURE REVIEWS. ENDOCRINOLOGY, NATURE PUBL. GROUP, US, [Online] vol. 14, no. 8, 29 June 2018 (2018-06-29), pages 476-500, XP037085262, ISSN: 1759-5029, DOI: 10.1038/S41574-018-0042-0 [retrieved on 2025-06-26] * abstract * * page 478; figure 1 * * page 487; figure 2 * * page 485, left-hand column, paragraph one - paragraph three * ----- -/-- | 1-13 | TECHNICAL FIELDS SEARCHED (IPC) |

The present search report has been drawn up for all claims

| Place of search | Date of completion of the search | Examiner |
|---|---|---|
| Munich | 2 July 2025 | Bradbrook, Derek |

EPO FORM 1503 03.82 (P04C01)

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ELLI F M ET AL: "Screening for GNAS genetic and epigenetic alterations in progressive osseous heteroplasia: First Italian series", BONE, PERGAMON PRESS., OXFORD, GB, vol. 56, no. 2, 21 June 2013 (2013-06-21), pages 276-280, XP028695915, ISSN: 8756-3282, DOI: 10.1016/J.BONE.2013.06.015 * the whole document * ----- | 1-13 | |

|  |  |  | **TECHNICAL FIELDS SEARCHED (IPC)** |
|---|---|---|---|

The present search report has been drawn up for all claims

| Place of search | Date of completion of the search | Examiner |
|---|---|---|
| Munich | 2 July 2025 | Bradbrook, Derek |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................................

& : member of the same patent family, corresponding document

EPO FORM 1503 03.82 (P04C01)

Europäisches
Patentamt
European
Patent Office
Office européen
des brevets

**Application Number**

EP 25 38 2052

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☒ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

34